# EUROPÄISCHE PATENTSCHRIFT

(11) **EP 3 650 212 B1**
(45) Veröffentlichungstag und Bekanntmachung des Hinweises auf die Patenterteilung: **03.04.2024**
(21) Anmeldenummer: 18205665.5
(22) Anmeldetag: 12.11.2018
(51) Int. Cl.: B30B 11/00, B30B 11/14, B30B 15/02, B30B 15/06, B30B 15/30, B30B 15/26

(54) **VERFAHREN ZUM AUTOMATISIERTEN HERSTELLEN VON INDIVIDUALISIERTEN TABLETTEN UND TABLETTENPRESSE ZUR AUTOMATISIERTEN HERSTELLUNG VON INDIVIDUALISIERTEN TABLETTEN**
METHOD FOR THE AUTOMATIC PRODUCTION OF INDIVIDUALIZED TABLETS AND TABLET PRESS FOR THE AUTOMATED PRODUCTION OF INDIVIDUALIZED TABLETS
PROCÉDÉ DE FABRICATION AUTOMATISÉE DE COMPRIMÉS INDIVIDUALISÉS ET PRESSE À COMPRIMÉS DESTINÉE À LA FABRICATION AUTOMATISÉE DE COMPRIMÉS INDIVIDUALISÉS

(43) Veröffentlichungstag der Anmeldung: 13.05.2020
(73) Patentinhaber: PrivMed X AB, 184 92 Åkersberga (SE)
(72) Erfinder: KRAUSE, Ingo, 184 92 Åkersberga (SE)
(74) Vertreter: Seemann & Partner Patentanwälte mbB

(56) Entgegenhaltungen:
- EP-A1- 2 977 195
- EP-A2- 2 823 958
- CN-U- 206 170 705
- US-A1- 2011 068 511
- US-A1- 2015 374 586
- US-A1- 2018 221 245

## Beschreibung

Die Erfindung betrifft ein Verfahren zum automatisierten Herstellen einer vorgebbaren Anzahl von individualisierten Tabletten mit einer vorgebbaren Menge eines ersten Wirkstoffs mittels einer Tablettenpresse.

Die Erfindung betrifft weiterhin eine Tablettenpresse zur automatisierten Herstellung einer vorgebbaren Anzahl von individualisierten Tabletten mit einer vorgebbaren Menge eines ersten Wirkstoffs.

Tabletten spielen heutzutage eine unverzichtbare Rolle in medizinischen und nicht medizinischen Bereichen, angefangen von der Verabreichung von Medikamenten bis hin zu Nahrungsergänzungsmitteln.

Zur Tablettenherstellung wird vielfach eine Matrize mit einer zentralen Öffnung eingesetzt, in die der Wirkstoff gefüllt wird, aus dem die Tablette hergestellt werden soll. Mittels eines Unterstempels und eines Oberstempels wird der Wirkstoff in der zentralen Öffnung zu einer Tablette gepresst.

Um den großen Bedarf an Tabletten zu decken, werden die Tabletten vielfach in standardisierten Größen mit einer festgelegten Menge an Wirkstoff produziert. Hierzu kommen beispielsweise Rundläuferpressen zum Einsatz, die bis zu hundert Matrizen und Stempelpaare umfassen, welche mittels einer Drehplatte rotiert und synchronisiert angetrieben werden. Solche Rundläuferpressen können bis zu 1,6 Millionen Tabletten pro Stunde herstellen.

Gerade bei medizinischen Anwendungen sind die zu verabreichenden Wirkstoffmengen jedoch abhängig von den individuellen Patienten, der Wirkstoffaufnahmefähigkeit und dem derzeitigen Gesundheitszustand dieser Patienten. Besonders, aber nicht nur, Kinder oder ältere Menschen bedürfen einer speziell abgestimmten Dosieranpassung.

Da gängige medizinische Wirkstoffpräparate meist nur in einer Anzahl von Standarddosierungen verfügbar sind, müssen Arzt und Apotheker fast immer eine Überdosierung wählen, da eine Unterdosierung nicht den angestrebten Behandlungserfolg hätte. Überdosierungen belasten jedoch das Organsystem der Patienten unnötig und verursachen auf Dauer Schäden. Das Aufteilen von Tabletten in kleinere Stücke mittels eines Messers ist für Patienten unpraktikabel und nicht zielführend.

Zudem werden Tabletten vielfach in Tablettenverpackungen mit einer vorgegebenen Anzahl an Tabletten verkauft. Dies führt zu einer Verschwendung von Pharmarohstoffen und überschüssigen Tabletten, die vom Patienten nicht benötigt und weggeworfen werden.

Aus US 2015/0374586 A1 ist ein Tablettenpressensystem zur Herstellung kleiner Mengen von Tabletten bekannt. Das System umfasst eine Matrize, in die ein Pulver eingefüllt wird, und eine Presse, mittels der das Pulver in der Matrize gepresst wird. Durch einen elektronischen Aktuator wird die Matrize zwischen verschiedenen Stationen des Systems hin und her bewegt.

US 2011/0068511 A1 zeigt eine Maschine zum Herstellen von Tabletten mittels Radiofrequenzenergie. Durch die Radiofrequenzenergie wird das eingefüllte Tablettenmaterial erhitzt, um die Tablettenstruktur zu verbessern. Es offenbart auch ein Verfahren zum automatisierten Herstellen einer vorgebbaren Anzahl von individualisierten Tabletten mit einer vorgebbaren Menge eines ersten Wirkstoffs mittels einer Tablettenpresse, die nur einen einzelnen Unterstempel umfasst, mit den folgenden Verfahrensschritten:
- Abfüllen einer Menge des ersten Wirkstoffs, die der Sollmasse entspricht, aus dem ersten Materialbehälter mittels der ersten Dosiervorrichtung in eine zentrale Öffnung einer Matrize, wobei eine Druckfläche des Unterstempels, deren Außenumfang einem Innenumfang der zentralen Öffnung entspricht, die zentrale Öffnung von unten verschließt,
- Pressen des ersten Wirkstoffs durch vertikales Auslenken einer Druckfläche des Oberstempels, deren Außenumfang dem Innenumfang der zentralen Öffnung entspricht, von oben in die zentrale Öffnung,
- Auslösen einer gepressten Tablette aus der Matrize durch Zurückfahren der Druckfläche des Oberstempels und vertikales Auslenken einer Druckfläche des Unterstempels von unten durch die zentrale Öffnung,
- Wiederholen der Verfahrensschritte des Abfüllens der Menge des ersten Wirkstoffs, des Pressens des ersten Wirkstoffs und des Auslösens der gepressten Tablette.

Es ist die Aufgabe der vorliegenden Erfindung, die für Patienten individualisierte Dosierung und Einnahme von in Tablettenform verabreichten Wirkstoffen zu vereinfachen.

Diese Aufgabe wird gelöst durch ein Verfahren zum automatisierten Herstellen einer vorgebbaren Anzahl von individualisierten Tabletten mit einer vorgebbaren Menge eines ersten Wirkstoffs mittels einer, insbesondere kompakten, Tablettenpresse, die nur einen einzelnen Unterstempel umfasst, mit den folgenden Verfahrensschritten:
- Eingabe einer Sollanzahl, die die Anzahl herzustellender Tabletten angibt, und einer Sollmasse, die die Menge des ersten Wirkstoffs pro Tablette angibt, in eine Eingabevorrichtung der Tablettenpresse,
- Übermitteln der Sollanzahl und der Sollmasse von der Eingabevorrichtung an ein Steuerungssystem der Tablettenpresse, wobei das Steuerungssystem eine erste Dosiervorrichtung eines ersten Materialbehälters, einen vertikal auslenkbaren Oberstempel und den vertikal auslenkbaren Unterstempel steuert,
- Abfüllen einer Menge des ersten Wirkstoffs, die der Sollmasse entspricht, aus dem ersten Materialbehälter mittels der ersten Dosiervorrichtung in eine zentrale Öffnung einer Matrize, wobei eine Druckfläche des Unterstempels, deren Außenumfang einem Innenumfang der zentralen Öffnung entspricht, die zentrale Öffnung von unten verschließt, wobei während des Abfüllens der Menge des ersten Wirkstoffs die erste Dosiervorrichtung mittels einer zweiten Positioniervorrichtung vertikal über der zentralen Öffnung angeordnet wird, wobei nach dem Abfüllen der Menge des ersten Wirkstoffs und vor dem Pressen des ersten Wirkstoffs der Oberstempel mittels der zweiten Positioniervorrichtung vertikal über der zentralen Öffnung angeordnet wird,
- Pressen des ersten Wirkstoffs durch vertikales Auslenken einer Druckfläche des Oberstempels, deren Außenumfang dem Innenumfang der zentralen Öffnung entspricht, von oben in die zentrale Öffnung,
- Auslösen einer gepressten Tablette aus der Matrize durch Zurückfahren der Druckfläche des Oberstempels und vertikales Auslenken einer Druckfläche des Unterstempels von unten durch die zentrale Öffnung,
- Transferieren der gepressten Tablette mittels der Transfervorrichtung in den Tablettenbehälter,
- Wiederholen der Verfahrensschritte des Abfüllens der Menge des ersten Wirkstoffs, des Pressens des ersten Wirkstoffs, und des Auslösens der gepressten Tablette, bis die Anzahl der gepressten Tabletten der Sollanzahl entspricht.

Insbesondere wird vor dem Verfahren ein Tablettenbehälter in einem Aufnahmeraum der Tablettenpresse angeordnet und nach dem Auslösen der gepressten Tablette mittels einer von dem Steuerungssystem gesteuerten Transfervorrichtung in den Tablettenbehälter transferiert.

Durch dieses Verfahren werden dem Patienten eine vorgebbare Sollanzahl individualisierte Tabletten mit einer vorgebbaren Sollmasse des ersten Wirkstoffs zur Verfügung gestellt. Die Sollmasse wird beispielsweise von einem Arzt oder einem Apotheker unter Berücksichtigung des Gesundheitszustands und der körperlichen Eigenschaften des Patienten, wie beispielsweise seinem Körpergewicht, festgelegt. Da die in der Tablette enthaltene Wirkstoffmenge exakt der Sollmasse entspricht, ist ein Zerteilen der Tablette überflüssig. Auf diese Weise werden vorteilhaft Überdosierungen vermieden und dem Patienten die Einnahme der Tabletten erleichtert.

Die Sollanzahl wird beispielsweise ebenfalls von dem Arzt oder dem Apotheker festgelegt. Dabei wird bevorzugt berücksichtigt, wie schnell sich der Gesundheitszustand des Patienten verändert und wie häufig eine Anpassung der Dosierung erforderlich wird. Die Sollanzahl wird bevorzugt so gewählt, dass die hergestellten Tabletten für die Behandlung des Patienten bis zur nächsten Diagnose ausreichen, ohne dass überflüssige Tabletten hergestellt werden. Eine Verschwendung von Tabletten durch die Abgabe von standardisierten Tablettenverpackungen, die mehr als die benötigten Tabletten enthalten, wird vorteilhaft vermieden.

Vorteilhaft ist die Tablettenpresse kompakt. Dies wird dadurch erreicht, dass die Tablettenpresse nur einen einzelnen Unterstempel, und insbesondere nur einen einzelnen Oberstempel, umfasst. Die Tablettenpresse befindet sich beispielsweise in einer Arztpraxis, einer Apotheke oder beim Patienten zu Hause. Bevorzugt werden die Tabletten direkt nach der Ermittlung der Sollmasse und der Sollanzahl in der Arztpraxis, in der Apotheke oder vom Patienten selbst bei sich zu Hause hergestellt. Es wird somit ein dezentrales Verfahren zur Herstellung von individualisierten Tabletten außerhalb von industriellen Fertigungsanlagen zur Verfügung gestellt. Da die Anzahl der von einem Patienten benötigen individualisierten Tabletten vergleichsweise klein ist, ist für ihre Herstellung keine hohe Fertigungsgeschwindigkeit notwendig. Stattdessen ist es vorteilhafterweise vorgesehen, dass eine Vielzahl von erfindungsgemäßen Tablettenpressen in einer Vielzahl von Arztpraxen, Apotheken oder Patientenwohnungen den Bedarf an individualisierten Tabletten der Patienten decken.

Der erste Materialbehälter ist insbesondere lösbar an einer ersten Dosieröffnung, insbesondere eines Pressraums, der Tablettenpresse fixiert. Dadurch lässt sich vorteilhaft der erste Materialbehälter schnell austauschen, beispielsweise wenn Tabletten mit einem anderen Wirkstoff hergestellt werden sollen. Die erste Dosiervorrichtung ist insbesondere ein Bestandteil des ersten Materialbehälters und bildet mit diesem eine Einheit. Der erste Wirkstoff ist entweder ein einzelner Wirkstoff oder eine Mischung von verschiedenen Wirkstoffen.

Insbesondere verschließt die Druckfläche des Unterstempels die zentrale Öffnung von unten, indem die Druckfläche des Unterstempels von unten in die zentrale Öffnung hineingeschoben wird. Dadurch entsteht eine Mulde, deren Wände von der zentralen Öffnung und deren Boden von der Druckfläche des Unterstempels gebildet werden. Zum Auslösen der gepressten Tablette wird die Druckfläche des Unterstempels weiter nach oben ausgelenkt, bis sie insbesondere plan mit der Oberkante der Matrize ist.

Die Eingabevorrichtung umfasst Eingabemittel zur Eingabe der Sollmasse und der Sollanzahl und ist insbesondere ein berührungsempfindlicher Bildschirm. Insbesondere ist die Eingabevorrichtung zudem dazu eingerichtet, auf einer Datenanzeige die eingegebene Sollanzahl und/oder die eingegebene Sollmasse und/oder zusätzliche Informationen, insbesondere Patientendaten und/oder Information über den ersten Wirkstoff, anzuzeigen. Weiterhin umfasst die Eingabevorrichtung ein Eingabemittel, mit dem die Herstellung der Tabletten gestartet wird. Insbesondere umfasst die Eingabevorrichtung ein Zugangssicherungssystem. Beispielsweise ist die Eingabevorrichtung mittels eines Zugangscodes gesichert oder umfasst einen Fingerabdruckscanner.

Insbesondere sind die Sollanzahl und die Sollmasse in einem Kennzeichen, insbesondere einem Strichcode codiert, das insbesondere auf einem dem Patienten ausgestellten Rezept aufgedruckt ist. Vorzugsweise wird das Kennzeichen mittels einer Bildaufnahmevorrichtung der Eingabevorrichtung, insbesondere einer Kamera, eingelesen und an das Steuerungssystem übermittelt, wobei das Steuerungssystem aus dem Kennzeichen die Sollanzahl und die Sollmasse bestimmt.

Das Steuerungssystem ist insbesondere ein Computer, der dazu eingerichtet ist, die in die Eingabevorrichtung eingegebene Sollmasse und Sollanzahl auszulesen und die erste Dosiervorrichtung, den Oberstempel, den Unterstempel und die Transfervorrichtung anzusteuern.

Die Transfervorrichtung umfasst insbesondere eine Schubvorrichtung, mittels derer die hergestellte Tablette von der Druckfläche des Unterstempels heruntergeschoben wird. Weiterhin insbesondere umfasst die Transfervorrichtung einen Kanal, der oberhalb eines in dem Aufnahmeraum angeordneten Tablettenbehälters mündet. Durch den Kanal fallen die von der Druckfläche des Unterstempels heruntergeschobenen Tabletten in den Tablettenbehälter. Direkt nach dem Transferieren der gepressten Tablette wird die nächste Tablette hergestellt, bis die Sollanzahl erreicht ist.

Bevorzugt berechnet das Steuerungssystem unter Berücksichtigung der Sollmasse, und gegebenenfalls einer Schüttdichte, des ersten Wirkstoffs ein Füllvolumen und damit einen Sollumfang, wählt aus wenigstens zwei in der Tablettenpresse angeordneten Matrizen mit unterschiedlichen Innenumfängen eine Sollmatrize aus, deren Innenumfang ihrer zentralen Öffnung dem Sollumfang entspricht, und steuert eine erste Positioniervorrichtung an, so dass die Sollmatrize über dem Unterstempel angeordnet wird, wobei insbesondere die erste Positioniervorrichtung eine zwischen dem Oberstempel und dem Unterstempel angeordnete erste Drehscheibe mit vertikaler Drehachse ist, an der die wenigstens zwei in der Tablettenpresse angeordneten Matrizen angeordnet, insbesondere fixiert, sind.

Vorteilhaft ist auf diese Weise die Größe der gepressten Tabletten variierbar. Das Steuersystem bestimmt dafür automatisch anhand der Sollmasse, und gegebenenfalls der Schüttdichte, des ersten Wirkstoffs ein Füllvolumen und damit einen passenden Sollumfang, wählt eine passende Sollmatrize aus und ordnet diese über dem Unterstempel an. Ein manueller Austausch der Matrize ist somit überflüssig. Durch die Variabilität der Größe der gepressten Tabletten lassen sich individualisierte Tabletten mit stark unterschiedlichen Wirkstoffmengen herstellen.

Insbesondere sind auf der Drehscheibe wenigstens drei Matrizen mit unterschiedlichen Innenumfängen ihrer zentralen Öffnungen fixiert.

Weiterhin bevorzugt wird der Außenumfang der Druckfläche des Oberstempels zum Pressen des ersten Wirkstoffs an den Innenumfang der Matrize angepasst, indem eine Anzahl von vertikal teleskopierend ineinander angeordneten Oberstempelschäften des Oberstempels nach unten ausgelenkt werden, wobei der Außenumfang des äußeren ausgelenkten Oberstempelschafts dem Innenumfang der Matrize entspricht.

Durch das Anpassen des Außenumfangs der Druckfläche des Oberstempels an den Innenumfang der Matrize wird vorteilhaft ein Auswechseln des Oberstempels beim Einsatz von Matrizen mit unterschiedlichen Innenumfängen überflüssig. Das Anpassen des Außenumfangs der Druckfläche des Oberstempels erfolgt durch das Auslenken der Oberstempelschäfte. Die Druckfläche des Oberstempels entspricht der Gesamtheit der Druckflächen der ausgelenkten Oberstempelschäfte. Insbesondere sind die Druckflächen der Oberstempelschäfte so ausgebildet, dass die Druckfläche des Oberstempels stets kontinuierlich ist.

Insbesondere umfasst der Oberstempel für jede auf der ersten Drehscheibe angeordnete Matrize einen zugeordneten Oberstempelschaft, dessen Querschnittsform der Querschnittsform der zentralen Öffnung einer der Matrizen entspricht. Insbesondere wird zum Pressen des ersten Wirkstoffs stets der der zentralen Öffnung zugeordnete Oberstempelschaft und alle weiter innen angeordneten Oberstempelschäfte ausgelenkt. Die Oberstempelschäfte sind insbesondere im Wesentlichen spaltfrei ineinander angeordnet. Auf diese Weise ist die Druckfläche des Oberstempels stets nutfrei, so dass Tabletten mit glatter Oberfläche hergestellt werden.

Vorzugsweise wird der Außenumfang der Druckfläche des Unterstempels vor dem Abfüllen der Menge des ersten Wirkstoffs an den Innenumfang der Matrize angepasst, indem eine Anzahl von vertikal teleskopierend ineinander angeordneten Unterstempelschäften des Unterstempels nach oben ausgelenkt werden, wobei der Außenumfang des äußeren ausgelenkten Unterstempelschafts dem Innenumfang der Matrize entspricht.

Durch das Anpassen des Außenumfangs der Druckfläche des Unterstempels an den Innenumfang der Matrizen wird vorteilhaft auch ein Auswechseln des Unterstempels beim Einsatz von Matrizen mit unterschiedlichen Innenumfängen überflüssig. Insbesondere wird der Außenumfang der Druckfläche des Unterstempels an den Innenumfang der Matrizen angepasst, bevor die Druckfläche des Unterstempels von unten in die zentrale Öffnung ausgelenkt wird, um diese von unten zu verschließen.

Die Unterstempelschäfte sind bevorzugt spiegelbildlich zu den Oberstempelschäften gefertigt, so dass die im Hinblick auf die Oberstempelschäfte beschriebenen Vorteile und Eigenschaften auch auf die Unterstempelschäfte zutreffen.

Die Masse des in die zentrale Öffnung abgefüllten ersten Wirkstoffes wird vorzugsweise gemessen, indem während des Abfüllens die Gesamtmasse des ersten Materialbehälters mittels einer Waage bestimmt und daraus die Menge des aus dem ersten Materialbehälter entnommenen ersten Wirkstoffs berechnet wird.

Die Masse des abgefüllten ersten Wirkstoffes wird mit anderen Worten mittels einer Differenzmessung der Restmasse des ersten Materialbehälters bestimmt. Dies erlaubt eine präzise Messung des abgefüllten ersten Wirkstoffs, da eine Messung im schwingungsanfälligen Pressraum überflüssig wird.

Während des Abfüllens der Menge des ersten Wirkstoffs wird die erste Dosiervorrichtung mittels einer zweiten Positioniervorrichtung vertikal über der zentralen Öffnung angeordnet, wobei nach dem Abfüllen der Menge des ersten Wirkstoffs und vor dem Pressen des ersten Wirkstoffs der Oberstempel mittels der zweiten Positioniervorrichtung vertikal über der zentralen Öffnung angeordnet wird, wobei insbesondere die zweite Positioniervorrichtung eine zweite Drehscheibe mit vertikaler Drehachse ist, an der die erste Dosiervorrichtung und der Oberstempel angeordnet, insbesondere fixiert, sind.

Durch die zweite Positioniervorrichtung wird vorteilhaft das Abfüllen des ersten Wirkstoffs erleichtert. Durch Anordnen der ersten Dosiervorrichtung über der zentralen Öffnung kann das Abfüllen des ersten Wirkstoffs direkt und verlustfrei von oben erfolgen. Nach dem Abfüllen des ersten Wirkstoffs wird die erste Dosiervorrichtung zur Seite bewegt, so dass der Oberstempel über der zentralen Öffnung angeordnet wird.

Weiterhin wird die Aufgabe gelöst durch ein Verfahren zum Herstellen einer vorgebbaren Anzahl individualisierter Zweischichttabletten mit einer vorgebbaren Menge des ersten Wirkstoffs und einer vorgebbaren Menge eines zweiten Wirkstoffs, wobei die Verfahrensschritte des zuvor beschriebenen Verfahrens mit folgenden zusätzlichen Verfahrensschritten ausgeführt werden:
- Eingeben einer Zweitschichtsollmasse in die Eingabevorrichtung und Übermitteln der Zweitschichtsollmasse an das Steuerungssystem, wobei die Zweischichtsollmasse die Menge des zweiten Wirkstoffs pro Zweischichttablette angibt,
- Positionieren einer zweiten Dosiervorrichtung eines zweiten Materialbehälters mittels der zweiten Positioniervorrichtung über der zentralen Öffnung,
- Abfüllen einer Menge eines zweiten Wirkstoffs, die der Zweitschichtsollmasse entspricht, mittels der zweiten Dosiervorrichtung in die zentrale Öffnung,
- Positionieren des Oberstempels mittels der zweiten Positioniervorrichtung über der zentralen Öffnung,
- Pressen des zweiten Wirkstoffs durch vertikales Auslenken der Druckfläche des Oberstempels von oben in die zentrale Öffnung,
wobei bis auf das Eingeben der Zweitschichtsollmasse und das Übermitteln der Zweitschichtsollmasse alle zusätzlichen Verfahrensschritte nach dem Pressen des ersten Wirkstoffs und vor dem Ausgeben der gepressten Tablette ausgeführt werden.

Vor der Durchführung dieses Verfahrens wird der zweite Materialbehälter, der mit dem zweiten Wirkstoff befüllt ist, an einer zweiten Dosieröffnung der Tablettenpresse und die zweite Dosiervorrichtung an einer freien Stelle der zweiten Positioniervorrichtung fixiert. Das Pressen des ersten Wirkstoffs erfolgt insbesondere mit einem kleineren Druck als das Pressen des zweiten Wirkstoffs. Auf diese Weise wird durch das Pressen des ersten Wirkstoffs eine Kavität für den zweiten Wirkstoff geschaffen und verhindert, dass die zwei Tablettenhemisphären nach dem Pressen des zweiten Wirkstoffs auseinanderfallen.

Durch dieses Verfahren lassen sich vorteilhaft individualisierte Zweischichttabletten herstellen, die an die Patientenbedürfnisse angepasst sind. Nach dem Herstellen einer ersten Schicht durch Abfüllen und Pressen des ersten Wirkstoffs wird mittels der zweiten Positioniervorrichtung die zweite Dosiervorrichtung über der zentralen Öffnung angeordnet und die zentrale Öffnung mit der Zweitschichtsollmasse des zweiten Wirkstoffs aufgefüllt. Anschließend wird mittels des Oberstempels der zweite Wirkstoff zu einer zweiten Schicht über der ersten Schicht gepresst. Anschließend wird die hergestellte Zweischichttablette auf die zuvor beschriebene Weise aus der Matrize ausgelöst und in den Tablettenbehälter transferiert.

Die Aufgabe wird außerdem gelöst durch eine Tablettenpresse zur automatisierten Herstellung einer vorgebbaren Anzahl von individualisierten Tabletten mit einer vorgebbaren Menge eines ersten Wirkstoffs, umfassend einen Pressraum, einen Aufnahmeraum zur Aufnahme eines Tablettenbehälters, einen mit dem ersten Wirkstoff befüllbaren ersten Materialbehälter, der eine erste Dosiervorrichtung aufweist, ein Steuerungssystem und eine Eingabevorrichtung, wobei der Pressraum einen vertikal auslenkbaren Oberstempel, einen vertikal auslenkbaren Unterstempel und wenigstens eine Matrize aufweist, wobei die Eingabevorrichtung Eingabemittel zur Eingabe einer Sollanzahl, die die Anzahl herzustellender Tabletten angibt, und einer Sollmasse, die die Menge des ersten Wirkstoffs pro Tablette angibt, umfasst, wobei die Eingabevorrichtung dazu eingerichtet ist, die Sollanzahl und die Sollmasse an das Steuerungssystem zu übermitteln, und wobei das Steuerungssystem dazu eingerichtet ist, die erste Dosiervorrichtung, den Oberstempel und den Unterstempel derart anzusteuern, dass eine Menge des ersten Wirkstoffs, die der Sollmasse entspricht, in eine zentrale Öffnung der Matrize gefüllt und mittels des Oberstempels und des Unterstempels zu einer Tablette gepresst wird und diesen Vorgang so oft zu wiederholen, bis die Sollanzahl an Tabletten hergestellt wurde, wobei die Tablettenpresse nur einen einzelnen Unterstempel umfasst, wobei eine zweite Positioniervorrichtung (80) umfasst ist, mittels derer wechselweise der Oberstempel (40) und die erste Dosiervorrichtung (12) vertikal über der zentralen Öffnung (60a, 61a, 62a) der über dem Unterstempel (50) angeordneten Matrize (60, 61, 62) anordbar sind.

Die Tablettenpresse verkörpert die gleichen Vorteile, Merkmale und Eigenschaften wie das zuvor beschriebene Verfahren zum automatisierten Herstellen einer vorgebbaren Anzahl von individualisierten Tabletten mit einer vorgebbaren Menge eines ersten Wirkstoffs mittels einer Tablettenpresse.

Vorteilhaft stellt die erfindungsgemäße Tablettenpresse eine dezentrale Vorrichtung zur Herstellung von individualisierten Tabletten dar. Die individualisierten Tabletten werden direkt vor Ort, also in einer Praxis, einer Apotheke oder einer Pharmaproduktion von fachkundigen Ärzten oder Apothekern oder von entsprechenden unterwiesenen Patienten hergestellt. Durch die individualisierten Tabletten werden Fehldosierungen/Überdosierungen vermieden und dem Patienten die Einnahme der Tabletten erleichtert, da diese nicht mehr zerteilt werden müssen.

Insbesondere ist der Unterstempel ortsfest im Pressraum angeordnet. Die Position des Unterstempels im Pressraum ändert sich somit nicht. Dadurch lässt sich die auf den Unterstempel wirkende Presskraft gut messen.

Die Tablettenpresse umfasst bevorzugt zwei Matrizen, deren zentrale Öffnungen unterschiedliche Innenumfänge aufweisen, wobei das Steuerungssystem dazu eingerichtet ist, unter Berücksichtigung der Sollmasse, und gegebenenfalls einer Schüttdichte, des ersten Wirkstoffs ein Füllvolumen und damit einen Sollumfang zu berechnen, aus den wenigstens zwei Matrizen eine Sollmatrize auszuwählen, deren Innenumfang ihrer zentralen Öffnung dem Sollumfang entspricht, und eine erste Positioniervorrichtung so anzusteuern, dass die Sollmatrize über dem Unterstempel angeordnet wird, wobei insbesondere die erste Positioniervorrichtung eine zwischen dem Oberstempel und dem Unterstempel angeordnete erste Drehscheibe mit vertikaler Drehachse ist, an der die wenigstens zwei Matrizen angeordnet, insbesondere fixiert, sind.

Vorzugsweise umfasst der Oberstempel wenigstens zwei vertikal teleskopierend ineinander angeordnete Oberstempelschäfte, mittels derer ein Außenumfang einer Druckfläche des Oberstempels durch individuelle Auslenkung der Oberstempelschäfte an den Innenumfang der zentralen Öffnung der wenigstens einen Matrize anpassbar ist.

Weiterhin vorzugsweise umfasst der Unterstempel wenigstens zwei vertikal teleskopierend ineinander angeordnete Unterstempelschäfte, mittels derer ein Außenumfang einer Druckfläche des Unterstempels durch individuelle Auslenkung der Unterstempelschäfte an den Innenumfang der zentralen Öffnung der wenigstens einen Matrize anpassbar ist.

Bevorzugt umfasst der Pressraum eine erste Schub- und Zugvorrichtung, mittels derer wenigstens ein oberer Arretierbolzen in eine horizontale Bohrung in Seitenwände der Oberstempelschäfte einschiebbar ist, um eine festlegbare Anzahl der Oberstempelschäfte vertikal zu fixieren, wobei insbesondere der Pressraum eine zweite Schub- und Zugvorrichtung umfasst, mittels derer wenigstens ein unterer Arretierbolzen in eine horizontale Bohrung in Seitenwände der Unterstempelschäfte einschiebbar ist, um eine festlegbare Anzahl der Unterstempelschäfte vertikal zu fixieren.

Mittels der Arretierbolzen wird erreicht, dass beim Auslenken des Oberstempels und des Unterstempels nur die gewünschten Stempelschäfte ausgelenkt werden.

Der Oberstempel wird insbesondere über einen oberen Kolben ausgelenkt, der von oben auf den Oberstempel drückt. Der Unterstempel wird insbesondere entsprechend über einen unteren Kolben ausgelenkt, der von unten auf den Unterstempel drückt. Insbesondere sind der obere Kolben ausschließlich mit dem innersten Oberstempelschaft und der untere Kolben ausschließlich mit dem innersten Unterstempelschaft verbunden. Sollen zusätzlich weitere Oberstempelschäfte und/oder Unterstempelschäfte ausgelenkt werden, werden diese weiteren Oberstempelschäfte und/oder Unterstempelschäfte mittels eines Koppelbolzens mit dem inneren Oberstempelschaft und/oder Unterstempelschaft gekoppelt. Der Koppelbolzen wird insbesondere von einer stabförmigen Schub- und Zugvorrichtung angetrieben. Die Schub- und Zugvorrichtung ist insbesondere in einer vertikal laufenden Nut innerhalb eines Stempelträgers geführt, so dass sie die vertikale Bewegung der ausgelenkten Oberstempelschäfte und/oder Unterstempelschäfte mitvollziehen kann.

Der erste Materialbehälter ist bevorzugt mit einer Waage gekoppelt, mittels derer die Gesamtmasse des ersten Materialbehälters messbar ist.

Es ist eine zweite Positioniervorrichtung umfasst, mittels derer wechselweise der Oberstempel und die erste Dosiervorrichtung, und insbesondere eine zweite Dosiervorrichtung eines zweiten Materialbehälters, vertikal über der zentralen Öffnung der über dem Unterstempel angeordneten Matrize anordbar sind, wobei insbesondere die zweite Positioniervorrichtung eine zweite Drehscheibe mit vertikaler Drehachse ist, an der der Oberstempel und die erste Dosiervorrichtung, und insbesondere die zweite Dosiervorrichtung, angeordnet, insbesondere fixiert, sind.

Gemäß einer bevorzugten Ausführungsform ist der Pressraum staubdicht, wobei insbesondere der Aufnahmeraum staubdicht verschließbar ist.

Vorteilhaft wird dadurch eine Kontamination des Aufnahmeraums und/oder eines in dem Aufnahmeraum angeordneten Tablettenbehälters und/oder des Bereichs außerhalb der Tablettenpresse vermieden.

Bevorzugt sind der Unterstempel und/oder der Oberstempel mit einer Druckmessvorrichtung gekoppelt, mittels derer ein auf den Unterstempel und/oder den Oberstempel wirkender Druck messbar ist.

Mittels der Druckmessvorrichtung lässt sich vorteilhaft der Druck während der Herstellung der Tabletten überwachen, eine Tablettenhärte und/oder Bruchfestigkeit der Tabletten steuern und eine Beschädigung der Stempel und/oder Matrizen verhindern.

Weitere Merkmale der Erfindung werden aus der Beschreibung erfindungsgemäßer Ausführungsformen zusammen mit den Ansprüchen und den beigefügten Zeichnungen ersichtlich.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: eine schematisch vereinfachte Darstellung einer Tablettenpresse zur automatisierten Herstellung einer vorgebbaren Anzahl von individualisierten Tabletten mit einer vorgebbaren Menge eines ersten Wirkstoffs,
- Fig. 2: eine schematisch vereinfachte Darstellung einer Eingabevorrichtung der Tablettenpresse aus Fig. 1,
- Fig. 3: eine schematisch vereinfachte Querschnittsansicht eines Pressraums und eines Materialbehälters einer Tablettenpresse während des Abfüllens eines Wirkstoffs,
- Fig. 4: eine schematisch vereinfachte Querschnittsansicht eines Pressraums und eines Materialbehälters einer Tablettenpresse während des Pressens eines Wirkstoffs,
- Fig. 5: eine schematisch vereinfachte Draufsicht auf eine erste Positioniervorrichtung,
- Fig. 6: eine schematisch vereinfachte Draufsicht auf eine zweite Positioniervorrichtung,
- Fig. 7a-d: schematisch vereinfachte Darstellungen eines Oberstempels mit teleskopierend ineinander angeordneten Oberstempelschäften und eines Unterstempels mit teleskopierend ineinander angeordneten Unterstempelschäften,
- Fig. 8a-c: schematisch vereinfachte Querschnittsansichten eines Oberstempels, eines Unterstempels und mehrerer Matrizen während des Pressvorgangs von Tabletten mit unterschiedlichen Wirkstoffmengen,
- Fig. 9a-c: schematisch vereinfachte Draufsichten auf einen Oberstempel während des Pressvorgangs von Tabletten mit unterschiedlichen Wirkstoffmengen.

In den Zeichnungen sind jeweils gleiche oder gleichartige Elemente und/oder Teile mit denselben Bezugsziffern versehen, so dass von einer erneuten Vorstellung jeweils abgesehen wird.

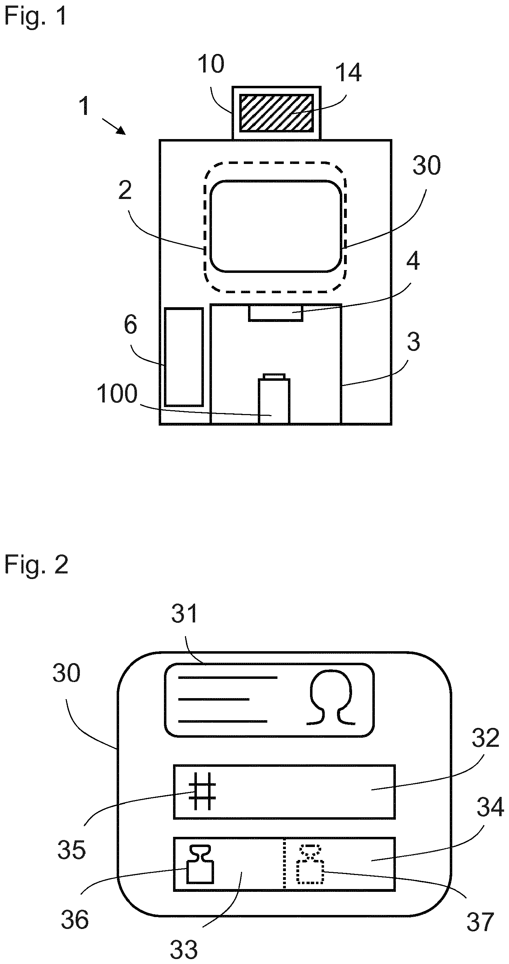

In Fig. 1 ist schematisch vereinfacht eine beispielhafte Ausführungsform einer erfindungsgemäßen Tablettenpresse 1 gezeigt. Die Tablettenpresse 1 umfasst einen Pressraum 2, dargestellt durch eine gestrichelte Linie, einen auf einer Hülle der Tablettenpresse 1 lösbar fixierten ersten Materialbehälter 10, der mit einem ersten Wirkstoff 14 gefüllt ist, einen Aufnahmeraum 3, in dem ein Tablettenbehälter 100 anordbar ist, eine Transfervorrichtung 4 zum Transport der hergestellten Tabletten aus dem Pressraum 2 in den Tablettenbehälter 100, ein Steuerungssystem 6 zur Steuerung der Tablettenpresse 1 und eine Eingabevorrichtung 30 zur Bedienung der Tablettenpresse 1. Insbesondere umfasst die Eingabevorrichtung 30 ein Zugangssicherungssystem. Beispielsweise ist die Eingabevorrichtung 30 mittels eines Zugangscodes gesichert oder umfasst einen Fingerabdruckscanner.

Das Steuerungssystem 6 ist beispielsweise ein Computer, der dazu eingerichtet ist, die Tablettenpresse 1 so zu steuern, dass automatisiert eine Sollanzahl von individualisierten Tabletten mit einer Sollmasse des ersten Wirkstoffs 14 hergestellt wird. Der Aufnahmeraum 3 lässt sich gemäß einer bevorzugten Ausführungsform verschlie-ßen, so dass während der Herstellung der Tabletten kein Staub aus der Tablettenpresse 1 austritt und keine Verunreinigung in die Tablettenpresse 1 gelangen.

Fig. 2 zeigt schematisch vereinfacht eine Detailansicht der Eingabevorrichtung 30, bei der es sich in der gezeigten Ausführungsform um einen berührungsempfindlichen Bildschirm (englisch: "Touchscreen") handelt. Die Eingabevorrichtung 30 umfasst eine Datenanzeige 31, beispielsweise ein Bereich des Bildschirms, auf der Informationen über den ersten Wirkstoff 14 und/oder einen Patienten angezeigt werden. Weiterhin umfasst die Eingabevorrichtung 30 mehrere Eingabemittel 32, 33, 34, bei denen es sich beispielsweise um weitere Bereiche des Bildschirms handelt. Mittels der Eingabemittel 32, 33, 34 lässt sich die Sollanzahl 35, die Sollmasse 36 und gegebenenfalls eine Zweitschichtsollmasse 37 eingeben. Die eingegebenen Werte werden zur Kontrolle angezeigt. Zudem ist ein nicht dargestelltes weiteres Eingabemittel vorgesehen, mit dem sich die Herstellung der Tabletten starten lässt.

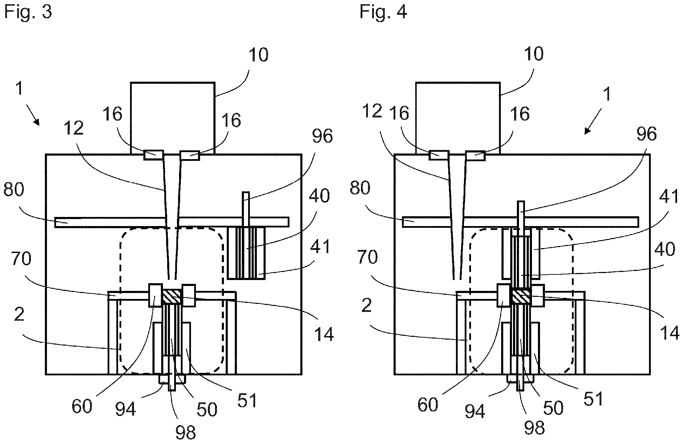

In Fig. 3 ist eine schematisch vereinfachte Querschnittsansicht der Tablettenpresse 1 mit dem Pressraum 2 und dem ersten Materialbehälter 10 gezeigt. Der erste Materialbehälter 10 umfasst eine erste Dosiervorrichtung 12 zum Abfüllen des ersten Wirkstoffs 14 in die zentrale Öffnung einer Matrize 60. Die erste Dosiervorrichtung 12 ist beispielsweise ein verschließbarer Trichter. Eine Waage 16 ist so angeordnet, dass sie die Gesamtmasse des ersten Materialbehälters 10 misst. Durch eine Differenzmessung der Masse des ersten Materialbehälters 10 vor und nach dem Abfüllen lässt sich die Masse des in die Matrize 60 abgefüllten ersten Wirkstoffs 14 bestimmen.

Die Matrize 60 ist auf einer ersten Positioniervorrichtung 70 innerhalb des Pressraums 2 fixiert, bei der es sich in der gezeigten Ausführungsform um eine erste Drehscheibe handelt. Auf dieser ersten Drehscheibe sind weitere, in Fig. 3 nicht dargestellte, Matrizen fixiert, welche sich durch Rotation der ersten Drehscheibe in eine Position oberhalb des Unterstempels 50 bewegen lassen. Dazu wird der Unterstempel 50 zunächst nach unten gefahren, damit sich die Drehscheibe rotieren lässt.

Unterhalb der Matrize 60 ist ein Unterstempel 50 im Pressraum 2 angeordnet. Der Unterstempel 50 ist mittels eines unteren Kolbens 98 in vertikaler Richtung auslenkbar. Ein Unterstempelträger 51 umfasst den Unterstempel 50 und stützt ihn ab. Mittels einer Druckmessvorrichtung 94 lässt sich der auf den Unterstempel 50 wirkende Druck messen. Vor dem Abfüllen des ersten Wirkstoffs 14 in die Matrize 60 wird der Unterstempel 50 von unten in die Matrize 60 bewegt, so dass eine Mulde zur Aufnahme des ersten Wirkstoffs 14 geschaffen wird.

In dem Pressraum 2 ist weiterhin ein Oberstempel 40 angeordnet, der durch einen Oberstempelträger 41 umfasst und abgestützt wird. Mittels eines oberen Kolbens 96 lässt sich der Oberstempel 40 in vertikaler Richtung auslenken.

Die erste Dosiervorrichtung 12 und der Oberstempel 40 sind an einer zweiten Positioniervorrichtung 80 fixiert, bei der es sich in der gezeigten Ausführungsform um eine zweite Drehscheibe handelt.

Zum Befüllen der Matrize 60 mit dem ersten Wirkstoff 14 wird die zweite Positioniervorrichtung 80 von dem Steuerungssystem 6 angesteuert, so dass die erste Dosiervorrichtung 12 über dem Unterstempel 50 und der Matrize 60 angeordnet wird, wie in Fig. 3 gezeigt. Dies geschieht natürlich nur, sofern die erste Dosiervorrichtung 12 noch nicht über dem Unterstempel 50 angeordnet ist. Anschließend wird die zentrale Öffnung der Matrize 60 mittels der ersten Dosiervorrichtung 12 mit dem ersten Wirkstoff 14 befüllt, bis die Sollmasse 36 erreicht ist. Dies wird mit der Waage 16 überwacht.

Nach dem Befüllen der Matrize 60 wird der erste Wirkstoff 14 gepresst. Dies ist in Fig. 4 gezeigt. Dazu wird erneut die zweite Positioniervorrichtung 80 von dem Steuerungssystem 6 angesteuert, so dass der Oberstempel 40 über dem Unterstempel 50 und der Matrize 60 angeordnet wird. Anschließend wird der Oberstempel 40 mittels des oberen Kolbens 96 von oben in die zentrale Öffnung der Matrize 60 ausgelenkt. Auf diese Weise wird der erste Wirkstoff 14 zwischen den Druckflächen des Unterstempels 50 und des Oberstempels 40 zu einer Tablette gepresst.

Nach dem Pressen der Tablette bewegen sich sowohl der Oberstempel 40 als auch der Unterstempel 50 nach oben, um die Tablette aus der Matrize 60 zu lösen. Anschließend wird die Tablette beispielsweise mittels einer nicht dargestellten Schubvorrichtung der Transfervorrichtung 4 von dem Unterstempel 50 heruntergeschoben, so dass sie durch einen Kanal der Transfervorrichtung 4 in den Tablettenbehälter 100 fällt.

Dieses Verfahren wird so lange wiederholt, bis die Sollanzahl 35 an Tabletten hergestellt wurde. Danach kann der Aufnahmeraum 3 geöffnet und der Tablettenbehälter 100 entnommen werden. Der Tablettenbehälter 100 ist nun mit der Sollanzahl 35 an individualisierten Tabletten mit der Sollmasse 36 des ersten Wirkstoffs 14 gefüllt.

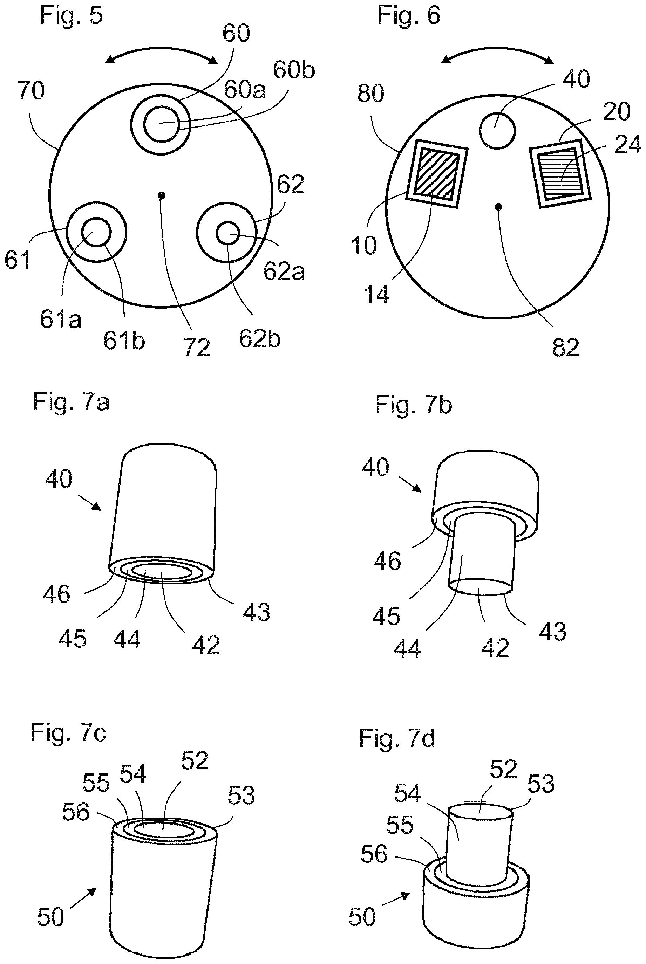

Fig. 5 zeigt eine schematisch vereinfachte Draufsicht auf eine beispielhafte erste Positioniervorrichtung 70. Diese ist in der dargestellten Ausführungsform als erste Drehscheibe mit vertikaler Drehachse 72 ausgeführt. An der ersten Drehscheibe sind drei Matrizen 60, 61, 62 fixiert. Die Matrizen 60, 61, 62 weisen jeweils eine zentrale Öffnung 60a, 61a, 62a mit unterschiedlichen, kreisförmigen Innenumfängen 60b, 61b, 62b auf. Die zentralen Öffnungen 60a, 61a, 62a reichen durch die erste Drehscheibe hindurch, so dass die Stempel 40, 50 durch die erste Drehscheibe nicht blockiert werden. Durch Rotation der ersten Drehscheibe lässt sich einstellen, welche der Matrizen 60, 61, 62 oberhalb des Unterstempels 50 angeordnet wird. Auf diese Weise lässt sich ein Sollumfang der hergestellten Tabletten einstellen, so dass Tabletten mit einer großen Bandbreite von Sollmassen 36 des ersten Wirkstoffs 14 mittels der Tablettenpresse 1 hergestellt werden können.

Alternativ sind statt der kreisförmigen Form der zentralen Öffnungen 60a, 61a, 62a ovale oder andere Formen vorgesehen, wodurch sich die Form der Tabletten anpassen lässt.

Fig. 6 zeigt eine schematisch vereinfachte Draufsicht auf die zweite Positioniervorrichtung 80. Diese ist in der dargestellten Ausführungsform als zweite Drehscheibe mit vertikaler Drehachse 82 ausgeführt. An der zweiten Drehscheibe ist der Oberstempel 40, der erste Materialbehälter 10 und, in der gezeigten Ausführungsform, ein zweiter Materialbehälter 20 fixiert, die sich durch Rotation der zweiten Drehscheibe wechselweise oberhalb des Unterstempels 50 anordnen lassen.

Der zweite Materialbehälter 20 ist mit einem zweiten Wirkstoff 24 zur Herstellung einer Zweischichttablette gefüllt. Dazu wird nach dem Pressen des ersten Wirkstoffs 14 mittels der zweiten Positioniervorrichtung 80 die zweite Dosiervorrichtung des zweiten Materialbehälters 20 über dem Unterstempel 50 angeordnet und die zentrale Öffnung 60a der auf diesem angeordneten Matrize 60 mit dem zweiten Wirkstoff 24 befüllt. Anschließend wird der Oberstempel 40 über den Unterstempel 50 bewegt und die Zweischichttablette gepresst.

Fig. 7a und 7b zeigen eine Ausführungsform eines teleskopierbaren Oberstempels 40. Der Oberstempel 40 umfasst drei teleskopierend ineinander angeordnete Oberstempelschäfte 44, 45, 46. In Fig. 7a sind alle drei Oberstempelschäfte 44, 45, 46 gemeinsam ausgelenkt, so dass der Außenumfang 43 der Druckfläche 42 des Oberstempels 40 dem Außenumfang des äußersten Oberstempelschafts 46 entspricht. In Fig. 7b ist ausschließlich der innerste Oberstempelschaft 44 ausgelenkt, so dass der Außenumfang 43 der Druckfläche 42 des Oberstempels 40 dem Außenumfang des innersten Oberstempelschafts 44 entspricht. Die Außenempfänge der Oberstempelschäfte 44, 45, 46 sind jeweils formkomplementär zu den Innenumfängen 60b, 61b, 62b der Matrizen 60, 61, 62.

Fig. 7c und 7d zeigen eine Ausführungsform eines teleskopierbaren Unterstempels 50, der spiegelbildlich zu dem Oberstempel 40 ausgebildet ist. Genau wie beim Oberstempel 40 lässt sich durch individuelle Auslenkung der Unterstempelschäfte 54, 55, 56 der Außenumfang 53 der Druckfläche 52 des Unterstempels 50 variieren.

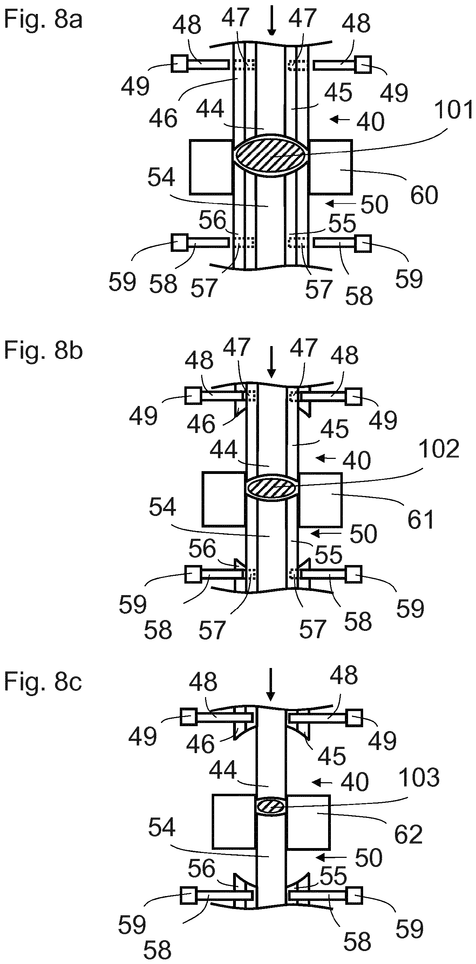

In Fig. 8a ist eine schematisch vereinfachte Querschnittsansicht eines teleskopierbaren Oberstempels 40, eines teleskopierbaren Unterstempels 50 und einer Matrize 60 während des Pressens einer großen Tablette 101 gezeigt. Dabei kommt die Matrize 60 mit der großen zentralen Öffnung 60a zum Einsatz. Zum Pressen der Tablette 101 werden sämtliche Oberstempelschäfte 44, 45, 46 und Unterstempelschäfte 54, 55, 56 genutzt. In den Oberstempelschäften 44, 45, 46 sind in der gezeigten Ausführungsform eine oder mehrere horizontale Bohrungen 47 vorgesehen, in die obere Arretierbolzen 48 mittels einer oder mehrerer schematisch angedeuteter erster Schub-und Zugvorrichtungen 49 einschiebbar sind. Analog sind in den Unterstempelschäften 54, 55, 56 in der gezeigten Ausführungsform eine oder mehrere horizontale Bohrungen 57 vorgesehen, in die untere Arretierbolzen 58 mittels einer oder mehrerer schematisch angedeuteter zweiter Schub-und Zugvorrichtungen 59 einschiebbar sind. Da die größte Matrize 60 eingesetzt wird, sind die Arretierbolzen 48, 58 so zurückgefahren, dass sie nicht in die horizontalen Bohrungen 47, 57 eingreifen. Die Schubrichtung des oberen Kolbens 96 ist durch einen Pfeil dargestellt.

Fig. 8b zeigt eine schematisch vereinfachte Querschnittsansicht während der Herstellung einer Tablette 102 mit mittlerer Größe. Dazu wird die Matrize 61 eingesetzt, die die zentrale Öffnung 61a mit mittlerer Größe aufweist. Die Arretierbolzen 48, 58 sind teilweise in die Bohrungen 47, 57 eingefahren, so dass die äußersten Stempelschäfte 46, 56 vertikal fixiert und lediglich die mittleren und die inneren Stempelschäfte 44, 45, 54, 55 ausgelenkt werden. Auf diese Weise werden die Außenumfänge 43, 53, der Druckflächen 42, 52 an den Innenumfang 61b der Matrize 61 angepasst.

Fig. 8c zeigt eine schematisch vereinfachte Querschnittsansicht während der Herstellung einer kleinen Tablette 103. Dazu wird die Matrize 62 eingesetzt, die die kleinste zentrale Öffnung 62a aufweist. Die Arretierbolzen 48, 58 sind vollständig in die Bohrungen 47, 57 eingefahren, so dass die äußersten und die mittleren Stempelschäfte 45, 46, 55, 56 vertikal fixiert und lediglich die inneren Stempelschäfte 44, 45, 54, 55 ausgelenkt werden. Auf diese Weise werden die Außenumfänge 43, 53, der Druckflächen 42, 52 an den Innenumfang 62b der Matrize 62 angepasst.

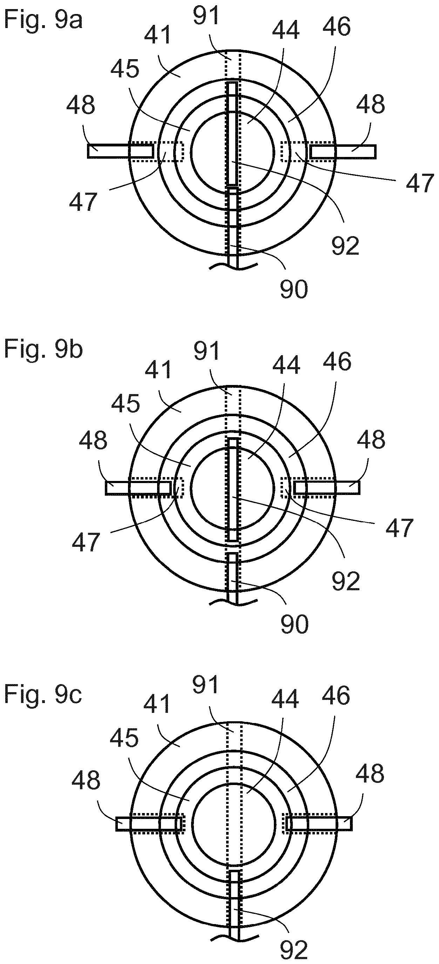

Fig. 9a zeigt eine schematisch vereinfachte Draufsicht von Fig. 8a mit zusätzlicher Darstellung des Oberstempelträgers 41 als äußerster Ring. Eine weitere horizontale Bohrung 91 ist senkrecht zu den Bohrungen 47 vorgesehen. In der Bohrung 91 wird ein Koppelbolzen 92 geführt, der mittels einer dritten Schub- und Zugvorrichtung 90 in der Bohrung 91 vor- und zurückgefahren wird.

Der obere Kolben 96 treibt in der in den Figuren 8a, 8b, 8c, 9a, 9b, 9c gezeigten Ausführungsform ausschließlich den inneren Oberstempelschaft 44 direkt an, während der untere Kolben 98 ausschließlich den inneren Unterstempelschaft 54 direkt antreibt. Mittels des Koppelbolzens 92 werden die Oberstempelschäfte 44, 45, 46 gegebenenfalls miteinander gekoppelt, so dass sie synchron auf und ab bewegt werden. Für den Unterstempel 50 ist dies analog vorgesehen. In Fig. 9a koppelt der Koppelbolzen 92 alle drei Oberstempelschäfte 44, 45, 46 miteinander, so dass diese zusammen vertikal ausgelenkt werden. Damit die dritte Schub- und Zugvorrichtung 90 nicht in dem Oberstempelträger 41 blockiert, ist in dem Oberstempelträger 41 eine vertikale Nut vorgesehen.

Fig. 9b zeigt eine schematisch vereinfachte Draufsicht von Fig. 8b.

Der Koppelbolzen 92 koppelt lediglich den inneren Oberstempelschaft 44 mit dem mittleren Oberstempelschaft 45, während der äu-ßere Oberstempelschaft 46 vertikal fixiert wird.

Fig. 9c zeigt eine schematisch vereinfachte Draufsicht von Fig. 8c. Der Koppelbolzen 92 ist eingefahren, so dass lediglich der innere Oberstempelschaft 44 ausgelenkt wird, während der mittlere Oberstempelschaft 45 und der äußere Oberstempelschaft 46 vertikal fixiert werden.

Im Rahmen der Erfindung sind Merkmale, die mit "insbesondere" oder "vorzugsweise" gekennzeichnet sind, als fakultative Merkmale zu verstehen.

### Bezugszeichenliste

- 1: Tablettenpresse
- 2: Pressraum
- 3: Aufnahmeraum
- 4: Transfervorrichtung
- 6: Steuerungssystem
- 10: erster Materialbehälter
- 12: erste Dosiervorrichtung
- 14: erster Wirkstoff
- 16: Waage
- 20: zweiter Materialbehälter
- 24: zweiter Wirkstoff
- 30: Eingabevorrichtung
- 31: Datenanzeige
- 32, 33, 34: Eingabemittel
- 35: Sollanzahl
- 36: Sollmasse
- 37: Zweitschichtsollmasse
- 40: Oberstempel
- 41: Oberstempelträger
- 42: Druckfläche
- 43: Außenumfang
- 44, 45, 46: Oberstempelschäfte
- 47: horizontale Bohrung
- 48: oberer Arretierbolzen
- 49: erste Schub- und Zugvorrichtung
- 50: Unterstempel
- 51: Unterstempelträger
- 52: Druckfläche
- 53: Außenumfang
- 54, 55, 56: Unterstempelschäfte
- 57: horizontale Bohrung
- 58: unterer Arretierbolzen
- 59: zweite Schub- und Zugvorrichtung
- 60, 61, 62: Matrize
- 60a, 61a, 62a: zentrale Öffnung
- 60b, 61b, 62b: Innenumfang
- 70: erste Positioniervorrichtung
- 72: Drehachse
- 80: zweite Positioniervorrichtung
- 82: Drehachse
- 90: dritte Schub- und Zugvorrichtung
- 91: horizontale Bohrung
- 92: Koppelbolzen
- 94: Druckmessvorrichtung
- 96: Oberer Kolben
- 98: Unterer Kolben
- 100: Tablettenbehälter
- 101, 102, 103: Tablette

## Patentansprüche

1. Verfahren zum automatisierten Herstellen einer vorgebbaren Anzahl von individualisierten Tabletten (101, 102, 103) mit einer vorgebbaren Menge eines ersten Wirkstoffs (14) mittels einer Tablettenpresse (1), die nur einen einzelnen Unterstempel (50) umfasst, mit den folgenden Verfahrensschritten:
- Eingabe einer Sollanzahl (35), die die Anzahl herzustellender Tabletten (101, 102, 103) angibt, und einer Sollmasse (36), die die Menge des ersten Wirkstoffs (14) pro Tablette (101, 102, 103) angibt, in eine Eingabevorrichtung (30) der Tablettenpresse (1),
- Übermitteln der Sollanzahl (35) und der Sollmasse (36) von der Eingabevorrichtung (30) an ein Steuerungssystem (6) der Tablettenpresse (1), wobei das Steuerungssystem (6) eine erste Dosiervorrichtung (12) eines ersten Materialbehälters (10), einen vertikal auslenkbaren Oberstempel (40) und den vertikal auslenkbaren Unterstempel (50) steuert,
- Abfüllen einer Menge des ersten Wirkstoffs (14), die der Sollmasse (36) entspricht, aus dem ersten Materialbehälter (14) mittels der ersten Dosiervorrichtung (12) in eine zentrale Öffnung (60a, 61a, 62a) einer Matrize (60, 61, 62), wobei eine Druckfläche (52) des Unterstempels (50), deren Außenumfang (53) einem Innenumfang (60b, 61b, 62b) der zentralen Öffnung (60a, 61a, 62a) entspricht, die zentrale Öffnung (60a, 61a, 62a) von unten verschließt, wobei während des Abfüllens der Menge des ersten Wirkstoffs (14) die erste Dosiervorrichtung (12) mittels einer zweiten Positioniervorrichtung (80) vertikal über der zentralen Öffnung (60a, 61a, 62a) angeordnet wird, wobei nach dem Abfüllen der Menge des ersten Wirkstoffs (14) und vor dem Pressen des ersten Wirkstoffs (14) der Oberstempel (40) mittels der zweiten Positioniervorrichtung (80) vertikal über der zentralen Öffnung (60a, 61a, 62a) angeordnet wird,
- Pressen des ersten Wirkstoffs (14) durch vertikales Auslenken einer Druckfläche (42) des Oberstempels (40), deren Außenumfang (42) dem Innenumfang (60b, 61b, 62b) der zentralen Öffnung (60a, 61a, 62a) entspricht, von oben in die zentrale Öffnung (60a, 61a, 62a),
- Auslösen einer gepressten Tablette (101, 102, 103) aus der Matrize (60, 61, 62) durch Zurückfahren der Druckfläche (42) des Oberstempels (40) und vertikales Auslenken einer Druckfläche (52) des Unterstempels (50) von unten durch die zentrale Öffnung (60a, 61a, 62a),
- Wiederholen der Verfahrensschritte des Abfüllens der Menge des ersten Wirkstoffs (14), des Pressens des ersten Wirkstoffs (14) und des Auslösens der gepressten Tablette (101, 102, 103), bis die Anzahl der gepressten Tabletten (101, 102, 103) der Sollanzahl (35) entspricht.

2. Verfahren nach Anspruch 1, **dadurch gekennzeichnet, dass** das Steuerungssystem (6) unter Berücksichtigung der Sollmasse (36), und insbesondere einer Schüttdichte, des ersten Wirkstoffs (14) ein Füllvolumen und damit einen Sollumfang berechnet, aus wenigstens zwei in der Tablettenpresse (1) angeordneten Matrizen (60, 61, 62) mit unterschiedlichen Innenumfängen (60b, 61b, 62b) eine Sollmatrize auswählt, deren Innenumfang (60b, 61b, 62b) ihrer zentralen Öffnung (60a, 61a, 62a) dem Sollumfang entspricht, und eine erste Positioniervorrichtung (70) ansteuert, so dass die Sollmatrize über dem Unterstempel (50) angeordnet wird, wobei insbesondere die erste Positioniervorrichtung (70) eine zwischen dem Oberstempel (40) und dem Unterstempel (50) angeordnete erste Drehscheibe mit vertikaler Drehachse (72) ist, an der die wenigstens zwei in der Tablettenpresse (1) angeordneten Matrizen (60, 61, 62) angeordnet, insbesondere fixiert, sind.

3. Verfahren nach Anspruch 1 oder 2, **dadurch gekennzeichnet, dass** der Außenumfang (43) der Druckfläche (42) des Oberstempels (40) zum Pressen des ersten Wirkstoffs (14) an den Innenumfang (60b, 61b, 62b) der Matrize (60, 61, 62) angepasst wird, indem eine Anzahl von vertikal teleskopierend ineinander angeordneten Oberstempelschäften (44, 45, 46) des Oberstempels (40) nach unten ausgelenkt werden, wobei der Außenumfang (43) des äußeren ausgelenkten Oberstempelschafts (44, 45, 46) dem Innenumfang (60b, 61b, 62b) der Matrize (60, 61, 62) entspricht.

4. Verfahren nach einem der Ansprüche 1 bis 3, **dadurch gekennzeichnet, dass** der Außenumfang (53) der Druckfläche (52) des Unterstempels (50) vor dem Abfüllen der Menge des ersten Wirkstoffs (14) an den Innenumfang (60b, 61b, 62b) der Matrize (60, 61, 62) angepasst wird, indem eine Anzahl von vertikal teleskopierend ineinander angeordneten Unterstempelschäften (54, 55, 56) des Unterstempels (50) nach oben ausgelenkt werden, wobei der Außenumfang (53) des äußeren ausgelenkten Unterstempelschafts (54, 55, 56) dem Innenumfang (60b, 61b, 62b) der Matrize (60, 61, 62) entspricht.

5. Verfahren nach einem der Ansprüche 1 bis 4, **dadurch gekennzeichnet, dass** die Masse des in die zentrale Öffnung (60a, 61a, 62a) abgefüllten ersten Wirkstoffes (14) gemessen wird, indem während des Abfüllens die Gesamtmasse des ersten Materialbehälters (10) mittels einer Waage (16) bestimmt und daraus die Menge des aus dem ersten Materialbehälter (10) entnommenen ersten Wirkstoffs (14) berechnet wird.

6. Verfahren nach einem der Ansprüche 1 bis 5, **dadurch gekennzeichnet, dass** die zweite Positioniervorrichtung (80) eine zweite Drehscheibe mit vertikaler Drehachse (82) ist, an der die erste Dosiervorrichtung (12) und der Oberstempel (40) angeordnet, insbesondere fixiert, sind.

7. Verfahren zum Herstellen einer vorgebbaren Anzahl individualisierter Zweischichttabletten mit einer vorgebbaren Menge des ersten Wirkstoffs (14) und einer vorgebbaren Menge eines zweiten Wirkstoffs (24), wobei die Verfahrensschritte nach Anspruch 6 mit folgenden zusätzlichen Verfahrensschritten ausgeführt werden:
- Eingeben einer Zweitschichtsollmasse (37) in die Eingabevorrichtung (30) und Übermitteln der Zweitschichtsollmasse (37) an das Steuerungssystem (6), wobei die Zweitschichtsollmasse (37) die Menge des zweiten Wirkstoffs (24) pro Zweischichttablette angibt,
- Positionieren einer zweiten Dosiervorrichtung eines zweiten Materialbehälters (20) mittels der zweiten Positioniervorrichtung (80) über der zentralen Öffnung (60a, 61a, 62a),
- Abfüllen einer Menge eines zweiten Wirkstoffs (24), die der Zweitschichtsollmasse (37) entspricht, mittels der zweiten Dosiervorrichtung in die zentrale Öffnung (60a, 61a, 62a),
- Positionieren des Oberstempels (40) mittels der zweiten Positioniervorrichtung (80) über der zentralen Öffnung (60a, 61a, 62a),
- Pressen des zweiten Wirkstoffs (14) durch vertikales Auslenken der Druckfläche (42) des Oberstempels (40) von oben in die zentrale Öffnung (60a, 61a, 62a),
wobei bis auf das Eingeben der Zweitschichtsollmasse (37) und das Übermitteln der Zweitschichtsollmasse (37) alle zusätzlichen Verfahrensschritte nach dem Pressen des ersten Wirkstoffs (14) und vor dem Ausgeben der gepressten Tablette (101, 102, 103) ausgeführt werden.

8. Tablettenpresse (1) zur automatisierten Herstellung einer vorgebbaren Anzahl von individualisierten Tabletten (101, 102, 103) mit einer vorgebbaren Menge eines ersten Wirkstoffs (14), umfassend einen Pressraum (2), einen Aufnahmeraum (3) zur Aufnahme eines Tablettenbehälters (100), einen mit dem ersten Wirkstoff (14) befüllbaren ersten Materialbehälter (10), der eine erste Dosiervorrichtung (12) aufweist, ein Steuerungssystem (6) und eine Eingabevorrichtung (30), wobei der Pressraum (2) einen vertikal auslenkbaren Oberstempel (40), einen vertikal auslenkbaren Unterstempel (50) und wenigstens eine Matrize (60, 61, 62) aufweist, wobei die Eingabevorrichtung (30) Eingabemittel (32, 33, 34) zur Eingabe einer Sollanzahl (35), die die Anzahl herzustellender Tabletten (101, 102, 103) angibt, und einer Sollmasse (36), die die Menge des ersten Wirkstoffs (14) pro Tablette (101, 102, 103) angibt, umfasst, wobei die Eingabevorrichtung (30) dazu eingerichtet ist, die Sollanzahl (35) und die Sollmasse (36) an das Steuerungssystem (6) zu übermitteln, und wobei das Steuerungssystem (6) dazu eingerichtet ist, die erste Dosiervorrichtung (12), den Oberstempel (40) und den Unterstempel (50) derart anzusteuern, dass eine Menge des ersten Wirkstoffs (14), die der Sollmasse (36) entspricht, in eine zentrale Öffnung (60a, 61a, 62a) der Matrize (60, 61, 62) gefüllt und mittels des Oberstempels (40) und des Unterstempels (40) zu einer Tablette (101, 102, 103) gepresst wird, und diesen Vorgang so oft zu wiederholen, bis die Sollanzahl (35) an Tabletten (101, 102, 103) hergestellt wurde, wobei die Tablettenpresse (1) nur einen einzelnen Unterstempel (50) umfasst, wobei eine zweite Positioniervorrichtung (80) umfasst ist, mittels derer wechselweise der Oberstempel (40) und die erste Dosiervorrichtung (12) vertikal über der zentralen Öffnung (60a, 61a, 62a) der über dem Unterstempel (50) angeordneten Matrize (60, 61, 62) anordbar sind.

9. Tablettenpresse (1) nach Anspruch 8, **dadurch gekennzeichnet, dass** die Tablettenpresse (1) wenigstens zwei Matrizen (60, 61, 62) umfasst, deren zentrale Öffnungen (60a, 61a, 62a) unterschiedliche Innenumfänge (60b, 61b, 62b) aufweisen, wobei das Steuerungssystem (6) dazu eingerichtet ist, unter Berücksichtigung der Sollmasse (36), und insbesondere einer Schüttdichte, des ersten Wirkstoffs (14) ein Füllvolumen und damit einen Sollumfang zu berechnen, aus den wenigstens zwei Matrizen (60, 61, 62) eine Sollmatrize auszuwählen, deren Innenumfang (60b, 61b, 62b) ihrer zentralen Öffnung (60a, 61a, 62a) dem Sollumfang entspricht, und eine erste Positioniervorrichtung (70) so anzusteuern, dass die Sollmatrize über dem Unterstempel (50) angeordnet wird, wobei insbesondere die erste Positioniervorrichtung (70) eine zwischen dem Oberstempel (40) und dem Unterstempel (50) angeordnete erste Drehscheibe mit vertikaler Drehachse (72) ist, an der die wenigstens zwei Matrizen (60, 61, 62) angeordnet, insbesondere fixiert, sind.

10. Tablettenpresse (1) nach Anspruch 8 oder 9, **dadurch gekennzeichnet, dass** der Oberstempel (40) wenigstens zwei vertikal teleskopierend ineinander angeordnete Oberstempelschäfte (44, 45, 46) umfasst, mittels derer ein Außenumfang (43) einer Druckfläche (42) des Oberstempels (40) durch individuelle Auslenkung der Oberstempelschäfte (44, 45, 46) an den Innenumfang (60b, 61b, 62b) der zentralen Öffnung (60a, 61a, 62a) der wenigstens einen Matrize (60, 61, 62) anpassbar ist.

11. Tablettenpresse (1) nach einem der Ansprüche 8 bis 10, **dadurch gekennzeichnet, dass** der Unterstempel (50) wenigstens zwei vertikal teleskopierend ineinander angeordnete Unterstempelschäfte (54, 55, 56) umfasst, mittels derer ein Außenumfang (53) einer Druckfläche (52) des Unterstempels (50) durch individuelle Auslenkung der Unterstempelschäfte (54, 55, 56) an den Innenumfang (60b, 61b, 62b) der zentralen Öffnung (60a, 61a, 62a) der wenigstens einen Matrize (60, 61, 62) anpassbar ist.

12. Tablettenpresse (1) nach Anspruch 10 oder 11, **dadurch gekennzeichnet, dass** der Pressraum (2) eine erste Schub- und Zugvorrichtung (49) umfasst, mittels derer wenigstens ein oberer Arretierbolzen (48) in eine horizontale Bohrung (47) in Seitenwände der Oberstempelschäfte (44, 45, 46) einschiebbar ist, um eine festlegbare Anzahl der Oberstempelschäfte (44, 45, 46) vertikal zu fixieren, wobei insbesondere der Pressraum (2) eine zweite Schub- und Zugvorrichtung (59) umfasst, mittels derer wenigstens ein unterer Arretierbolzen (58) in eine horizontale Bohrung (57) in Seitenwände der Unterstempelschäfte (54, 55, 56) einschiebbar ist, um eine festlegbare Anzahl der Unterstempelschäfte (54, 55, 56) vertikal zu fixieren.

13. Tablettenpresse (1) nach einem der Ansprüche 8 bis 12, **dadurch gekennzeichnet, dass** der erste Materialbehälter (10) mit einer Waage (16) gekoppelt ist, mittels derer die Gesamtmasse des ersten Materialbehälters (10) messbar ist.

14. Tablettenpresse (1) nach einem der Ansprüche 8 bis 13, **dadurch gekennzeichnet, dass** mittels der zweiten Positioniervorrichtung (80) eine zweite Dosiervorrichtung eines zweiten Materialbehälters (20) vertikal über der zentralen Öffnung (60a, 61a, 62a) der über dem Unterstempel (50) angeordneten Matrize (60, 61, 62) anordbar sind, wobei insbesondere die zweite Positioniervorrichtung (80) eine zweite Drehscheibe mit vertikaler Drehachse (82) ist, an der der Oberstempel (40) und die erste Dosiervorrichtung (12), und insbesondere die zweite Dosiervorrichtung, angeordnet, insbesondere fixiert, sind.

15. Tablettenpresse (1) nach einem der Ansprüche 8 bis 14, **dadurch gekennzeichnet, dass** der Unterstempel (50) und/oder der Oberstempel (40) mit einer Druckmessvorrichtung (94) gekoppelt sind, mittels derer ein auf den Unterstempel (50) und/oder den Oberstempel (40) wirkender Druck messbar ist.

## Claims

1. Method for automatically producing a specifiable number of individualized tablets (101, 102, 103) with a specifiable quantity of a first active substance (14) using a tablet press (1), which comprises only a single lower punch (50), the method comprising:
- inputting a target number (35), which indicates the number of tablets (101, 102, 103) to be produced and a target mass(36), which indicates the quantity of the first active substance (14) per tablet (101, 102, 103) into an input device (30) of the tablet press (1),
- transmitting the target number (35) and the target mass (36) from the input device (30) to a control system (6) of the tablet press (1), wherein the control system (6) is configured to control the tablet press (1), wherein the control system (1) controls a first dosing device (12) of a first material container (10), a vertically extendable upper punch (40), and the vertically extendable lower punch (50),
- filling a quantity of the first active substance (14), which corresponds to the target mass (36), from the first material container (14) into a central opening (60a, 61a, 62a) of a die (60, 61, 62) using the first dosing device (12), wherein a pressure face (52) of the lower punch (50), an outer circumference (53) of which corresponds to an inner circumference (60b, 61b, 62b) of the central opening (60a, 61a, 62a), closes the central opening (60a, 61a, 62a) from below, wherein the first dosing device (12) is arranged vertically above the central opening (60a, 61a, 62a) by a second positioning device (80) during the filling of the quantity of the first active substance (14), wherein the upper punch (40) is arranged vertically above the central opening (60a, 61a, 62a) by the second positioning device (80) after the filling of the quantity of the first active substance (14) and before the pressing of the first active substance (14),
- pressing the first active substance (14) by vertically extending a pressure face (42) of the upper punch (40), an outer circumference (42) of which corresponds to the inner circumference (60b, 61b, 62b) of the central opening (60a, 61a, 62a), into the central opening (60a, 61a, 62a) from above,
- releasing a pressed tablet (101, 102, 103) from the die (60, 61, 62) by retracting the pressure face (42) of the upper punch (40) and vertically extending a pressure face (52) of the lower punch (50) through the central opening (60a, 61a, 62a) from below,
- repeating the filling of the quantity of the first active substance (14), the pressing of the first active substance (14), and the releasing of the pressed tablet (101, 102, 103) until the number of pressed tablets (101, 102, 103) corresponds to the target number (35).

2. The method according to claim 1, **characterized in that** the control system (6) is configured to calculate a filling volume and a target circumference based on the target mass (36), and in particular a bulk density, of the first active substance (14), select a target die from at least two dies (60, 61, 62) arranged in the tablet press (1) with different inner circumferences (60b, 61b, 62b), the inner circumference (60b, 61b, 62b) of the central opening (60a, 61a, 62a) of the target die corresponding to the target circumference, and actuate a first positioning device (70) such that the target die is arranged above the lower punch (50), wherein in particular the first positioning device (70) is a first rotary disk with a vertical axis of rotation (72) arranged between the upper punch (40) and the lower punch (50), on which the at least two dies (60, 61, 62) arranged in the tablet press (1) are arranged, in particular fixed.

3. The method according to claim 1 or 2, **characterized in that** the outer circumference (43) of the pressure face (42) of the upper punch (40) for pressing the first active substance (14) is adapted to the inner circumference (60b, 61b, 62b) of the die (60, 61, 62) **in that** a number of vertically telescoping upper punch shafts (44, 45, 46) of the upper punch (40) arranged inside each other are extended downwards, wherein the outer circumference (43) of the outer extended upper punch shaft (44, 45, 46) corresponds to the inner circumference (60b, 61b, 62b) of the die (60, 61, 62).

4. The method according to one of the claims 1 to 3, **characterized in that** the outer circumference (53) of the pressure face (52) of the lower punch (50) is adapted to the inner circumference (60b, 61b, 62b) of the die (60, 61, 62) before the filling of the quantity of the first active substance (14) in that a number of vertically telescoping lower punch shafts (54, 55, 56) of the lower punch (50) arranged inside each other are extended upwards, wherein the outer circumference (53) of the outer extended lower punch shaft (54, 55, 56) corresponds to the inner circumference (60b, 61b, 62b) of the die (60, 61, 62).

5. The method according to one of the claims 1 to 4, **characterized in that** the mass of the first active substance (14) filled into the central opening (60a, 61a, 62a) is measured such that, during the filling, the total mass of the first material container (10) is determined by a scale (16) and from this the quantity of the first active substance (14) taken from the first material container (10) is calculated.

6. The method according to one of the claims 1 to 5, **characterized in that** the second positioning device (80) is a second rotary disk with a vertical axis (82) of rotation, on which the first dosing device (12) and the upper punch (40) are arranged, in particular fixed.

7. Method for producing a specifiable number of individualized two-layer tablets with a specifiable quantity of the first active substance (14) and a specifiable quantity of a second active substance (24), wherein the method steps according to claim 6 are carried out with the following additional method steps.
- inputting a second-layer target mass (37) into the input device (30) and transmitting the second-layer target mass (37) to the control system (6), wherein the second-layer target mass (37) indicates a quantity of the second active substance (24) per two-layer tablet,
- positioning a second dosing device of a second material container (20) above the central opening (60, 61a, 62a) by the second positioning device (80),
- filling a quantity of the second active substance (24) that corresponds to the second-layer target mass (37) into the central opening (60, 61a, 62a) by the second dosing device,
- positioning the upper punch (40) above the central opening (60, 61a, 62a) by the second positioning device (80),
- pressing the second active substance (14) by vertically extending the pressure face (42) of the upper punch (40) into the central opening (60, 61a, 62a) from above,
wherein, except for inputting the second-layer target mass (37) and transmitting the second-layer target mass (37), all additional method steps are carried out after pressing the first active substance (14) and before dispensing the pressed tablet (101, 102, 103).

8. Tablet press (1) for automatically producing a specifiable number of individualized tablets (101, 102, 103) with a specifiable quantity of a first active substance (14), comprising a press chamber (2), a receiving chamber (3) for receiving a tablet container (100), a first material container (10) fillable with the first active substance (14) and comprising a first dosing device (12), a control system (6), and an input device (30), wherein the press chamber (2) has a vertically extendable upper punch (40), a vertically extendable lower punch (50), and at least one die (60, 61, 62), wherein the input device (30) comprises input means (32, 33, 34) for inputting a target number (35) that indicates the number of tablets (101, 102, 103) to be produced and a target mass (36) that indicates the quantity of the first active substance (14) per tablet (101, 102, 103), wherein the input device (30) is configured to transmit the target number (35) and the target mass (36) to the control system (6), and wherein the control system (6) is configured to actuate the first dosing device (12), the upper punch (40) and the lower punch (50) such that a quantity of the first active substance (14) that corresponds to the target mass (36) is filled into a central opening (60a, 61a, 62a) of the die (60, 61, 62) and pressed by the upper punch (40) and the lower punch (50) in order to form a tablet (101, 102, 103), and to repeat this process until the target number (35) of tablets (101, 102, 103) are produced, wherein the tablet press (1) comprises only a single lower punch (50), wherein a second positioning device (80) is comprised, by means of which the upper punch (40) and the first dosing device (12) can be arranged alternately vertically above the central opening (60a, 61a, 62a) of the die (60, 61, 62) arranged above the lower punch (50).

9. Tablet press (1) according to claim 8, **characterized in that** the tablet press (1) comprises at least two dies (60, 61, 62), the central openings (60a, 61a, 62a) of which have different inner circumferences (60b, 61b, 62b), wherein the control system (6) is configured to calculate a filling volume and thus a target circumference based on the target mass (36), and in particular a bulk density, of the first active substance (14), to select a target die from the at least two dies (60, 61, 62), the inner circumference (60b, 61b, 62b) of its central opening (60a, 61a, 62a) corresponding to the target circumference, and to actuate a first positioning device (70) such that the target die is arranged above the lower punch (50), wherein in particular the first positioning device (70) is a first rotary disk with a vertical axis of rotation (72) arranged between the upper punch (40) and the lower punch (50), on which the at least two dies (60, 61, 62) are arranged, in particular fixed.

10. Tablet press (1) according to claim 8 or 9, **characterized in that** the upper punch (40) comprises at least two vertically telescoping upper punch shafts (44, 45, 46) arranged inside each other, an outer circumference (43) of a pressure face (42) of the upper punch (40) being adapted to the inner circumference (60b, 61b, 62b) of the central opening (60a, 61a, 62a) of the at least one die (60, 61, 62) by individually extending the upper punch shafts (44, 45, 46).

11. Tablet press (1) according to one of the claims 8 to 10, **characterized in that** the lower punch (50) comprises at least two vertically telescoping lower punch shafts (54, 55, 56) arranged inside each other, an outer circumference (53) of a pressure face (52) of the lower punch (50) being adapted to the inner circumference (60b, 61b, 62b) of the central opening (60a, 61a, 62a) of the at least one(60, 61, 62) die by individually extending the lower punch shafts (44, 45, 46).

12. Tablet press (1) according to claim 10 or 11, **characterized in that** the press chamber (2) comprises a first pushing and pulling device (49), by means of which at least one upper locking pin (48) is pushable into a horizontal hole (47) in side walls of the upper punch shafts (44, 45, 46) to vertically fix a definable number of the upper punch shafts (44, 45, 46), wherein in particular the press chamber (2) comprises a second pushing and pulling device (59) , by means of which at least one lower locking pin (48) is pushable into a horizontal hole (57) in side walls of the lower punch shafts (54, 55, 56) to vertically fix a definable number of the lower punch shafts (54, 55, 56).

13. Tablet press (1) according to one of the claims 8 to 12, **characterized in that** the first material container (10) is coupled with a scale (16), by means of which total mass of the first material container (10) is measurable.

14. Tablet press (1) according to one of the claims 8 to 13, **characterized in that** by means of the second positioning device (80) a second dosing device of a second material container (20) can be arranged vertically above the central opening (60a, 61a, 62a) of the die (60, 61, 62) arranged above the lower punch (50), wherein in particular the second positioning device (80) is a second rotary disk with a vertical axis of rotation (82), on which the upper punch (40) and the first dosing device (12), and in particular the second dosing device, are arranged, in particular fixed.

15. Tablet press (1) according to one of the claims 8 to 14, wherein the lower punch (50) and/or the upper punch (40) are coupled with a pressure measuring device (94), by means of which a pressure acting on the lower punch (50) and/or the upper punch (40) is measurable.

## Revendications

1. Procédé de fabrication automatisée d'un nombre prédéterminable de comprimés individualisés (101, 102, 103) avec une quantité prédéterminable d'un premier principe actif (14), au moyen d'une presse à comprimés (1) qui ne comprend qu'un seul poinçon inférieur (50), comprenant les étapes de procédé suivantes :
- saisie d'un nombre cible (35), qui indique le nombre de comprimés (101, 102, 103) à fabriquer, et d'une masse cible (36), qui indique la quantité du premier principe actif (14) par comprimé (101, 102, 103), dans un dispositif d'entrée (30) de la presse à comprimés (1),
- transmission du nombre cible (35) et de la masse cible (36) du dispositif d'entrée (30) à un système de commande (6) de la presse à comprimés (1), le système de commande (6) commandant un premier dispositif de dosage (12) d'un premier récipient de matière (10), un poinçon supérieur (40) apte à être dévié verticalement et le poinçon inférieur (50) apte à être dévié verticalement,
- remplissage d'une quantité du premier principe actif (14), qui correspond à la masse cible (36), à partir du premier récipient de matière (14) au moyen du premier dispositif de dosage (12) dans une ouverture centrale (60a, 61a, 62a) d'une matrice (60, 61, 62), une surface de pression (52) du poinçon inférieur (50), dont la périphérie extérieure (53) correspond à une périphérie intérieure (60b, 61b, 62b) de l'ouverture centrale (60a, 61a, 62a), fermant l'ouverture centrale (60a, 61a, 62a) par le bas ; pendant le remplissage de la quantité du premier principe actif (14), le premier dispositif de dosage (12) est agencé verticalement au-dessus de l'ouverture centrale (60a, 61a, 62a) au moyen d'un deuxième dispositif de positionnement (80) ; après le remplissage de la quantité du premier principe actif (14) et avant le pressage du premier principe actif (14), le poinçon supérieur (40) est agencé verticalement au-dessus de l'ouverture centrale (60a, 61a, 62a) au moyen du deuxième dispositif de positionnement (80),
- pressage du premier principe actif (14) dans l'ouverture centrale (60a, 61a, 62a) depuis le haut, en déviant verticalement une surface de pression (42) du poinçon supérieur (40), dont la périphérie extérieure (42) correspond à la périphérie intérieure (60b, 61b, 62b) de l'ouverture centrale (60a, 61a, 62a),
- libération d'un comprimé pressé (101, 102, 103) hors de la matrice (60, 61, 62) à travers l'ouverture centrale (60a, 61a, 62a), par le bas, par recul de la surface de pression (42) du poinçon supérieur (40) et en déviant verticalement une surface de pression (52) du poinçon inférieur (50),
- répétition des étapes du processus consistant à introduire la quantité du premier principe actif (14), à presser le premier principe actif (14) et à libérer le comprimé pressé (101, 102, 103) jusqu'à ce que le nombre de comprimés pressés (101, 102, 103) corresponde au nombre cible (35).

2. Procédé selon la revendication 1, **caractérisé en ce que** le système de commande (6) calcule un volume de remplissage, et donc une périphérie cible, en tenant compte de la masse cible (36), et en particulier d'une densité apparente, du premier principe actif (14), sélectionne, parmi au moins deux matrices (60, 61, 62) agencées dans la presse à comprimés (1) et ayant des périphéries intérieures (60b, 61b, 62b) différentes, une matrice cible dont la périphérie intérieure (60b, 61b, 62b) de son ouverture centrale (60a, 61a, 62a) correspond à la périphérie cible, et commande un premier dispositif de positionnement (70) de sorte que la matrice cible soit agencée au-dessus du poinçon inférieur (50), le premier dispositif de positionnement (70) étant en particulier un premier disque rotatif (72) à axe de rotation vertical agencé entre le poinçon supérieur (40) et le poinçon inférieur (50), sur lequel sont agencées, en particulier fixées, lesdites au moins deux matrices (60, 61, 62) agencées dans la presse à comprimés (1).

3. Procédé selon la revendication 1 ou la revendication 2, **caractérisé en ce que** la périphérie extérieure (43) de la surface de pression (42) du poinçon supérieur (40) pour presser le premier principe actif (14) est adaptée à la périphérie intérieure (60b, 61b, 62b) de la matrice (60, 61, 62), en déviant vers le bas un certain nombre de tiges de poinçon supérieur (44, 45, 46) du poinçon supérieur (40) agencées verticalement de manière télescopique les unes dans les autres, la périphérie extérieure (43) de la tige de poinçon supérieur extérieure déviée (44, 45, 46) correspondant à la périphérie intérieure (60b, 61b, 62b) de la matrice (60, 61, 62).

4. Procédé selon l'une des revendications 1 à 3, **caractérisé en ce que** le périmètre extérieur (53) de la surface de pression (52) du poinçon inférieur (50) est adapté au périmètre intérieur (60b, 61b, 62b) de la matrice (60, 61, 62) avant le remplissage de la quantité du premier principe actif (14), en déviant vers le haut un certain nombre de tiges de poinçon inférieur (54, 55, 56) du poinçon inférieur (50), agencées verticalement de manière télescopique les unes dans les autres, la périphérie extérieure (53) de la tige de poinçon inférieur extérieure déviée (54, 55, 56) correspondant à la périphérie intérieure (60b, 61b, 62b) de la matrice (60, 61, 62).

5. Procédé selon l'une des revendications 1 à 4, **caractérisé en ce que** la masse du premier principe actif (14) versé dans l'ouverture centrale (60a, 61a, 62a) est mesurée en déterminant, pendant le remplissage, la masse totale du premier récipient de matière (10) au moyen d'une balance (16) et en calculant, à partir de cette masse, la quantité du premier principe actif (14) prélevée dans le premier récipient de matière (10).

6. Procédé selon l'une des revendications 1 à 5, **caractérisé en ce que** le deuxième dispositif de positionnement (80) est un deuxième plateau tournant à axe de rotation vertical (32), sur lequel sont agencés, notamment fixés, le premier dispositif de dosage (12) et le poinçon supérieur (40).

7. Procédé de fabrication d'un nombre prédéterminable de comprimés à deux couches individualisés avec une quantité prédéterminable du premier principe actif (14) et une quantité prédéterminable d'un deuxième principe actif (24), les étapes du procédé selon la revendication 6 étant mises en œuvre avec les étapes de procédé supplémentaires suivantes :
- saisir une masse cible de deuxième couche (37) dans le dispositif d'entrée (30) et transmettre la masse cible de deuxième couche (37) au système de commande (6), la masse cible de deuxième couche (37) indiquant la quantité du deuxième principe actif (24) par comprimé à deux couches,
- positionner un deuxième dispositif de dosage d'un deuxième récipient de matière (20) au moyen du deuxième dispositif de positionnement (80) au-dessus de l'ouverture centrale (60a, 61a, 62a),
- remplir dans l'ouverture centrale (60a, 61a, 62a) une quantité d'un deuxième principe actif (24) correspondant à la masse cible de deuxième couche (37) au moyen du deuxième dispositif de dosage,
- positionner le poinçon supérieur (40) au-dessus de l'ouverture centrale (60a, 61a, 62a) au moyen du deuxième dispositif de positionnement (80),
- presser le deuxième principe actif (14) dans l'ouverture centrale (60a, 61a, 62a), par le haut, en déviant verticalement la surface de pression (42) du poinçon supérieur (40),
toutes les étapes supplémentaires du procédé, à l'exception de la saisie de la masse cible de la deuxième couche (37) et de la transmission de la masse cible de la deuxième couche (37), étant mises en oeuvre après le pressage du premier principe actif (14) et avant la distribution du comprimé pressé (101, 102, 103).

8. Presse à comprimés (1) pour la fabrication automatisée d'un nombre prédéterminable de comprimés individualisés (101, 102, 103) avec une quantité prédéterminable d'un premier principe actif (14), comprenant une chambre de compression (2), une chambre de réception (3) pour recevoir un récipient à comprimés (100), un premier récipient de matière (10) apte à être introduit avec le premier principe actif (14), qui présente un premier dispositif de dosage (12), un système de commande (6) et un dispositif d'entrée (30), la chambre de compression (2) présentant un poinçon supérieur (40) apte à être dévié verticalement, un poinçon inférieur (50) apte à être dévié verticalement et au moins une matrice (60, 61, 62), le dispositif d'entrée (30) présentant des moyens de saisie (32, 33, 34) pour saisir un nombre cible (35), qui indique le nombre de comprimés (101, 102, 103) à fabriquer, et une masse cible (36) qui indique la quantité du premier principe actif (14) par comprimé (101, 102, 103), le dispositif d'entrée (30) étant configuré de façon à transmettre le nombre cible (35) et la masse cible (36) au système de commande (6), le système de commande (6) étant conçu de façon à commander le premier dispositif de dosage (12), le poinçon supérieur (40) et le poinçon inférieur (50) de telle manière qu'une quantité du premier principe actif (14), qui correspond à la masse cible (36), est remplie dans une ouverture centrale (60a, 61a, 62a) de la matrice (60, 61, 62) et est pressée en un comprimé (101, 102, 103) au moyen du poinçon supérieur (40) et du poinçon inférieur (40), et à répéter ce processus jusqu'à ce que le nombre cible (35) de comprimés (101, 102, 103) ait été produit, la presse à comprimés (1) ne comprenant qu'un seul poinçon inférieur (50), un deuxième dispositif de positionnement (80) étant inclus, au moyen duquel le poinçon supérieur (40) et le premier dispositif de dosage (12) sont aptes à être agencés alternativement verticalement au-dessus de l'ouverture centrale (60a, 61a, 62a) de la matrice (60, 61, 62) agencée au-dessus du poinçon inférieur (50).

9. Presse à comprimés (1) selon la revendication 8, **caractérisée en ce que** la presse à comprimés (1) comprend au moins deux matrices (60, 61, 62) dont les ouvertures centrales (60a, 61a, 62a) présentent des périphéries intérieures (60b, 61b, 62b) différentes, le système de commande (6) étant conçu pour calculer un volume de remplissage, et donc une périphérie cible, en tenant compte de la masse cible (36), et en particulier d'une densité en vrac, du premier principe actif (14), pour choisir parmi lesdites au moins deux matrices (60, 61, 62) une matrice cible dont la périphérie intérieure (60b, 61b, 62b) de son ouverture centrale (60a, 61a, 62a) correspond à la périphérie cible, et pour commander un premier dispositif de positionnement (70) de telle sorte que la matrice cible soit agencée au-dessus du poinçon inférieur (50), le premier dispositif de positionnement (70) étant en particulier un premier disque rotatif à axe de rotation vertical (72) agencé entre le poinçon supérieur (40) et le poinçon inférieur (50), sur lequel sont agencées, en particulier fixées, lesdites au moins deux matrices (60, 61, 62).

10. Presse à comprimés (1) selon la revendication 8 ou la revendication 9, **caractérisée en ce que** le poinçon supérieur (40) comprend au moins deux tiges de poinçon supérieur (44, 45, 46) agencées verticalement de manière télescopique l'une dans l'autre, au moyen desquelles une périphérie extérieure (43) d'une surface de pression (42) du poinçon supérieur (40) est apte à être adaptée à la périphérie intérieure (60b, 61b, 62b) de l'ouverture centrale (60a, 61a, 62a) de ladite au moins une matrice (60, 61, 62) par une déviation individuelle des tiges de poinçon supérieur (44, 45, 46).

11. Presse à comprimés (1) selon l'une des revendications 8 à 10, **caractérisée en ce que** le poinçon inférieur (50) comprend au moins deux tiges de poinçon inférieur (54, 55, 56) agencées verticalement de manière télescopique l'une dans l'autre, au moyen desquelles une périphérie extérieure (53) d'une surface de pression (52) du poinçon inférieur (50) est apte à être adaptée à la périphérie intérieure (60b, 61b, 62b) de l'ouverture centrale (60a, 61a, 62a) de ladite au moins une matrice (60, 61, 62) par déviation individuelle des tiges de poinçon inférieur (54, 55, 56).

12. Presse à comprimés (1) selon la revendication 10 ou la revendication 11, **caractérisée en ce que** la chambre de compression (2) comprend un premier dispositif de poussée et de traction (49), au moyen duquel au moins un pêne de verrouillage supérieur (48) est apte à être inséré dans un alésage horizontal (47) dans des parois latérales des tiges de poinçon supérieur (44, 45, 46), afin de fixer un nombre déterminable de tiges de poinçon supérieur (44, 45, 46), la chambre de compression (2) comprenant en particulier un deuxième dispositif de poussée et de traction (59), au moyen duquel au moins un pêne de verrouillage inférieur (58) est apte à être inséré dans un alésage horizontal (57) dans des parois latérales des tiges de poinçon inférieur (54, 55, 56), afin de fixer verticalement un nombre déterminable des tiges de poinçon inférieur (54, 55, 56).

13. Presse à comprimés (1) selon l'une des revendications 8 à 12, **caractérisée en ce que** le premier récipient de matière (10) est couplé à une balance (16) au moyen de laquelle la masse totale du premier récipient de matière (10) est apte à être mesurée.

14. Presse à comprimés (1) selon l'une des revendications 8 à 13, **caractérisée en ce qu'**au moyen du deuxième dispositif de positionnement (80), un deuxième dispositif de dosage d'un deuxième récipient de matière (20) est placé verticalement au-dessus de l'ouverture centrale (60a, 61a, 62a) de la matrice (60, 61, 62), le deuxième dispositif de positionnement (80) étant notamment un deuxième disque rotatif (82) à axe de rotation vertical, sur lequel sont agencés, notamment fixés, le poinçon supérieur (40) et le premier dispositif de dosage (12), et notamment le deuxième dispositif de dosage.

15. Presse à comprimés (1) selon l'une des revendications 8 à 14, **caractérisée en ce que** le poinçon inférieur (50) et/ou le poinçon supérieur (40) sont couplés à un dispositif de mesure de pression (94), au moyen duquel une pression agissant sur le poinçon inférieur (50) et/ou une pression agissant sur le poinçon supérieur (40) sont aptes à être mesurées.
